Europäisches Patentamt

European Patent Office

Office européen des brevets

(11) Publication number: **0 198 386**
**A2**

# EUROPEAN PATENT APPLICATION

(21) Application number: 86104742.1

(22) Date of filing: **07.04.86**

(51) Int. Cl.⁴: **C12N 15/00 , C12N 5/00**

(30) Priority: 05.04.85 US 720092
03.10.85 US 783743

(43) Date of publication of application:
**22.10.86 Bulletin 86/43**

(84) Designated Contracting States:
**AT BE CH DE FR GB IT LI LU NL SE**

(71) Applicant: **MELOY LABORATORIES, INC.**
**6715 Electronic Drive**
**Springfield Virginia 22151(US)**

(72) Inventor: **Sarver, Nava**
**8122 Tuckerman Lane**
**Potomac Maryland(US)**
Inventor: **Ricca, George A.**
**7903 Foote Lane**
**Springfield Virginia(US)**
Inventor: **Drohan, William N.**
**5232 Perth Court**
**Springfield Virginia(US)**

(74) Representative: **Patentanwälte Grünecker,**
**Kinkeldey, Stockmair & Partner**
**Maximilianstrasse 58**
**D-8000 München 22(DE)**

(54) Sustained high-level production of recombinant human gamma interferon using a bovine papillomavirus vector.

(57) This invention provides a new Bovine Papillomavirus (BPV) vector capable of replication in both prokaryotic and eukaryotic hosts, capable of directing the expression and export of a variety of heterologous protein products including gamma interferon (IFN-γ).

A process for producing (IFN-γ) employing hosts transformed by the vector is also disclosed.

## SUSTAINED HIGH-LEVEL PRODUCTION OF RECOMBINANT HUMAN GAMMA INTERFERON USING A BOVINE PAPILLOMAVIRUS VECTOR

This invention relates to a process for producing recombinant human immune interferon. More particularly, the invention relates to a process for production of recombinant human immune interferon in mammalian cell expression systems using a bovine papillomavirus shuttle vector.

Interferon is a glycoprotein whose synthesis in cells is principally induced by viruses or mitogens. Interferons are classified into three major species designated IFN-$\alpha$ (leukocyte), IFN-$\beta$ (fibroblast), and IFN-$\gamma$ (immune). Interferons possess potent antiviral, anticellular, immunoregulatory, and antitumor activities. Interferon treatment of cancer and viral infections in animals has met with some success. Consequently, immune interferon is of great interest clinically as well as academically.

In general, recombinant DNA techniques have now become well known. See Methods In Enzymology, (Academic Press) Volumes 65 and 68 - (1979); 100 and 101 (1983) and the references cited therein, all of which are incorporated herein by reference. An extensive technical discussion embodying most commonly used recombinant DNA methodologies can be found in Maniatis, et al., Molecular Cloning, Cold Spring Harbor Laboratory (1982). Genes coding for various polypeptides may be cloned by incorporating a DNA fragment coding for the polypeptide in a recombinant DNA vehicle, e.g., bacterial or viral vectors, followed by transformation of a suitable host. Previously, this host was primarily an Escherichia coli (E. coli) cell line. However, with the development of eukaryotic viral vectors, it is now possible to reintroduce cloned genes into their natural environment where mechanisms involved in control of gene expression and developmental regulations can be tested. An important application of eukaryotic vectors is the production of biologically active gene products suitable for pharmacological use.

Several methods are currently in use for delivering defined foreign deoxyribonucleic acid - (DNA) segments into eukaryotic cells. These include physical injection of DNA [Mueller, C., A. Graessmann, and M. Graessmann, Cell (1978) 15:579], fusion of DNA containing liposomes - [Dimitriadis, G.J., Nature (London) (1978) 274 :923)] or erythrocytes [Rechsteiner, M., Natl. Cancer Inst. Monogr. (1978) 48 :57] with target cells, and the direct application of naked DNA onto cells in the presence of calcium phosphate [Graham, F.L., and A.J. van der Eb. Virology (1973) 52:456] and marker DNA [Mantei, N., B. Werner and C. Weissmann, Nature, (London) (1979) 281:40; M. Wigler, et al., Cell (1977) 11:223; and B. Wold, et al., Proc. Natl. Acad. Sci. USA (1979), 76:5684].

More recently, insertion of DNA into recipient cells was achieved by using viral particles (Simian virus 40 [SV40] in which a segment of the viral genome is covalently linked to defined nucleic acid segments [Gruss, P., and G. Khoury, Proc. Natl. Acad. Sci. (1981) 78:133; Hamer, D.H., and P. Leder, Nature (1979), 281:35-40; and R.C. Mulligan, et al., Nature (London) (1979) 277:108]. Whereas the SV40 vector system offers a rapid and efficient way to introduce foreign DNAs into permissive host cells, the system is limited by the size of DNA that can be accomodated within the virus particle. Moreover, since monkey cells are permissive for SV40 replication, infection by recombinant SV40 particles culminates in cell death. SV40 DNA has not yet been exploited as a cloning vector in non-permissive rodent cells, because (i) SV40 transformation is associated with integration of the viral genome, a process that may disrupt the integrity of the foreign DNA segment of interest, and (ii) there is no indication that the gene will be active at detectable levels from the low integrated copy numbers which are sufficient for the expression of the SV40-transforming gene.

The use of bovine papillomavirus (BPV) DNA as a viral vector is well documented and was originally based on the observation that the viral genome persists as an extrachromosomal plasmid in transformed cells (Law, M.-F., D.R. Lowy, I. Dvoretzky, and P.M. Howley, Proc. Natl. Acad. Sci., USA 78:2727:2731, 1981). In this form, the cloned gene is maintained in a uniform sequence environment of the BPV minichromosome eliminating potential problems associated with the integration of the cloned DNA into inactive regions of the host chromosome. This property was fully exploited in the establishment of BPV shuttle vectors capable of replicating in prokaryote and eukaryotic cells - [Sarver, N., J.C. Byrne, and P.M. Howley, Proc. Natl. Acad. Sci. USA, 79(29):7147-7151, 1982; DiMaio, D., R. Treisman, and T. Maniatis, Proc. Natl. Acad. Sci. USA, 79(13):4030-4034, 1982; and Kushner, P.J., B.B. Levinson, and H.M. Goodman, J. Mol. Appln. Genet., 1 (6):527-528, 1982]. Such vectors are efficient in directing the regulated expression from inducible promoters and in expressing gene products destined for intra and extracellular location.

Vectors which may be employed as starting materials useful for the construction of the vectors of the subject invention are described in U.S. Patent 4,419,446, the contents of which are incorporated herein by reference.

Another improvement in the BPV vector system was the incorporation of a dominant selectable marker which has expanded the range of cells capable of supporting amplified BPV plasmid replication beyond those manifesting the transformed phenotype. This invention realizes the utility of BPV DNA to imortalize cells and to exist as multicopy plasmid in transduced cells. It further describes adaptation of these transduced cells for prolonged amplified expression of a foreign gene using human gamma IFN cDNA as the model gene.

The present invention relates to a bovine papillomavirus expression vector capable of replication in a prokaryotic host and replication and expression of non-native genetic information in an eukaryotic host comprising a prokaryotic promoter, a eukaryotic promoter, at least two dominant selectable markers, a signal sequence, and an enhancer element.

The present invention has made it possible to provide readily available large quantities of human immune interferon. This has been achieved by the application of recombinant DNA technology to preparing cloning vehicles encoding for the human immune interferon protein, and by screening/ isolating procedures for recovering human immune interferon essentially free of other proteins of human origin.

Accordingly, the present invention provides human immune interferon or its fragments essentially free of other proteins of human origin. Characteristically, the immune interferon protein is glycosylated. Immune interferon is produced by recombinant DNA techniques in host cells or other self-replicating systems and is provided in essentially pure form. Also provided are methods and compositions for preparing the above-described interferon as well as therapeutic compositions and uses for the interferon protein or fragments in the treatment of cancer or viral infections in humans and animals.

The invention further provides replicable expression vectors incorporating a DNA sequence encoding immune interferon and a self-replicating host cell system transformed or transfected thereby. The host system is mammalian cells.

The human immune interferon is produced by a process which comprises (a) preparing a replicable expression vector capable of expressing the DNA sequence encoding interferon in a suitable host cell system; (b) transforming said host system to obtain a recombinant host system; (c) maintaining said recombinant host system under conditions permitting expression of said interferon encoding DNA sequence to produce immune interferon protein; and (d) recovering said interferon protein.

Preferably, the interferon-encoding replicable expression vector is made by preparing a double-stranded complementary DNA (ds-cDNA) preparation representative of interferon mRNA and incorporating the ds-cDNA into replicable expression vectors within a eukaryotic transcription cassette containing regulatory elements required for transcription of said cDNA. The preferred mode of recovering the interferon comprises reacting the proteins expressed by the recombinant host system with a reagent composition comprising at least one binding protein specific for interferon. Binding proteins such as antibodies to IFN-γ, either monoclonal or polyclonal, would be useful in the recovery step. See for example the antibody described by Lee J., et al. (J. Immunol. Methods 69(1) 61-70 (1984)).

According to one embodiment, the invention provides a bovine papillomavirus expression vector capable of dual host replication by virtue of the presence of a prokaryotic replicon and a eukaryotic replicon. The vector also contains a eukaryotic promoter, a prokaryotic promoter, at least two dominant selectable markers, and a signal sequence.

According to a further embodiment, this invention provides various derivatives of BPV expression vectors characterized in possessing increased expression capabilities when compared to a standard vector.

According to another embodiment, the invention provides a host transformed by a bovine papillomavirus expression vector capable of replication in a prokaryotic replicon, a eukaryotic replicon, a eukaryotic promoter, at least two dominant selectable markers, and a signal sequence.

According to a final embodiment, the invention provides a process for producing human interferon comprising providing a replicable expression vector capable of expressing the DNA sequence encoding immune interferon in a suitable host, transforming said host, and maintaining said transformed host under conditions permitting the expression of said interferon, the improvement which comprises the employing the vector pdBV-γ -IFN as the replicable expression vector.

DETAILED DESCRIPTION OF THE DRAWINGS

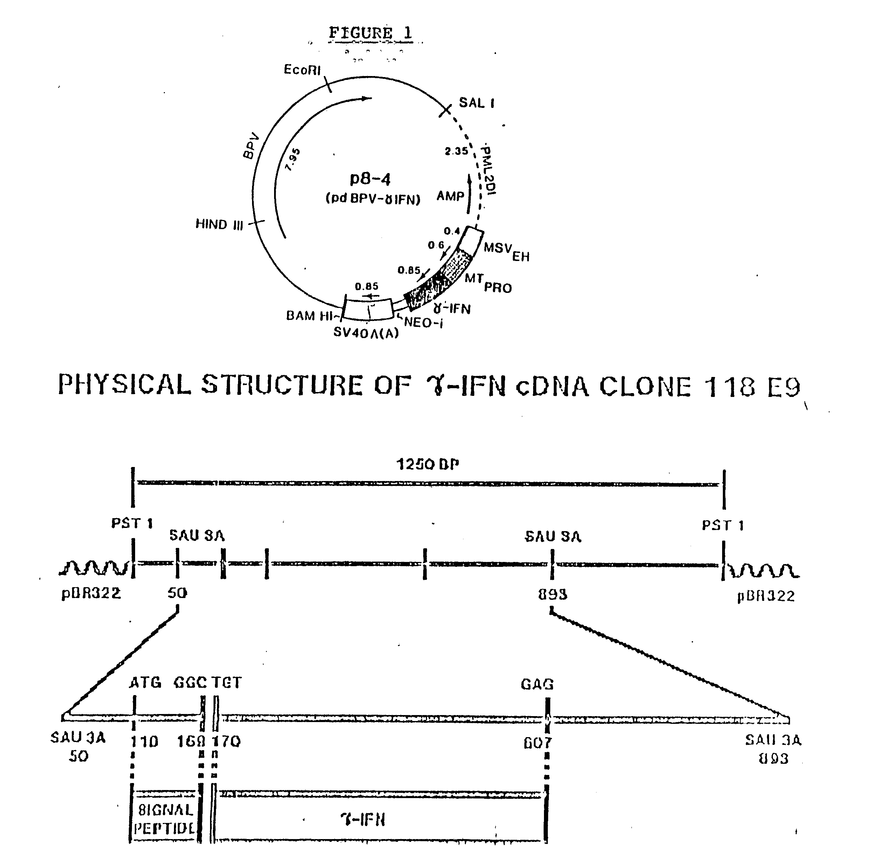

Figure 1 illustrates the physical map of pdBPV-human gamma interferon (p8-4) DNA (top). The human gamma interferon cDNA insert is shown in greater detail (bottom).

The plasmid consists of the following structural elements described in a clockwise direction. The complete BPV genome is joined to the pML2dl sequence containing the pBR322 origin of replication and a functional beta-lactamase gene. The moloney murine sarcoma virus enhancer (MSV) was originally derived from the proviral LTR as a HinfI-XbaI fragment (141-525, respectively). Both termini had previously been converted to BamH1 sites with synthetic linkers. The mouse metallothionein I promoter is a KpnI to Bg1II fragment of approximately 600 bp. The KpnI site was previously modified to a Bg1II site to facilitate cloning. Downstream from the promoter is the human gamma-interferon cDNA obtained as a Sau3A fragment and is followed by a non-functional 3' region of the neomycin gene. The 3' processing signals are contained within an 850 bp fragment derived from SV40 DNA. A Sau3A fragment (843 bp) consists of the complete coding sequence for human gamma-interferon including the signal peptide and is shown in detail at the bottom of Figure 1. The 5' and 3' untranslated regions contain 60 bp and 286 bp, respectively.

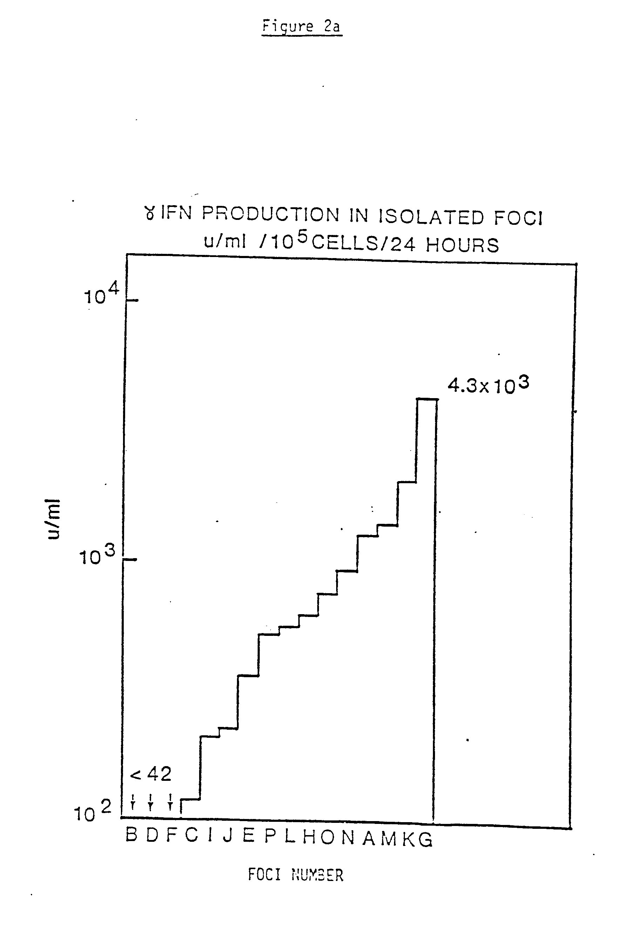

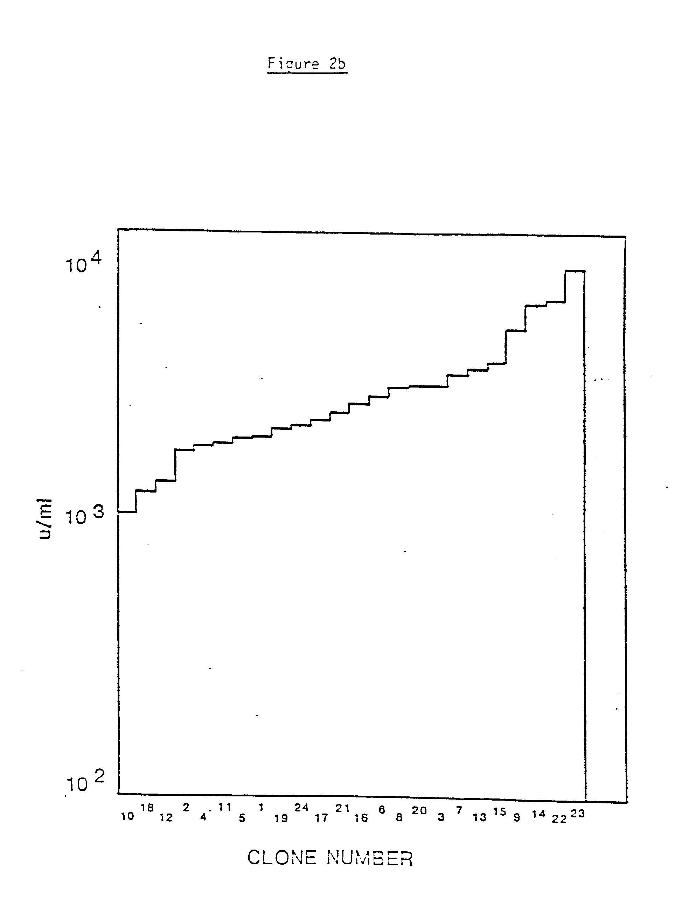

Figures 2A and 2B illustrate the expression levels of recombinant γ-IFN in isolated foci and in single cell clones of transformed C127 cells. DNA transfections were performed using the calcium phosphate coprecipitation method followed by enhancement with 25% dimethylsulfoxide as described previously. Phenotypically transformed foci were isolated and established as cell lines. Single cell cloning was achieved by plating at limited dilution into 96 well plates, visual identification of wells containing a single cell, and propagation of these cells into established lines. Cells were maintained in either complete medium (Dulbecco modified Eagle medium supplemented with 100 U/ml penicillin, 100 ug/ml streptomycin, and 10% heat inactivated fetal calf serum) or in defined medium lacking fetal calf serum (medium 199 containing 10 ug/ml insulin, 10 ug/ml transferrin, 10 ng/ml EGF, 2 ng/ml BSA, 150 ug/ml bovine brain extract, 1.4 uM hydrocortisone, and 1 nM 3, 3', 5-triiodo-D-thyronine). γ-IFN activity was assayed by a cytopathic effect inhibition assay using 3-fold serial dilutions of samples on human WISH cells challanged with encephalomyocarditis virus (EMC).

A: Expression levels in 16 isolated foci (NS8-4-A -NS8-4-P) derived from p8-4 transformed C127 cells.

B: Expression levels in 23 single cell clones derived from parental line NS8-4-G.

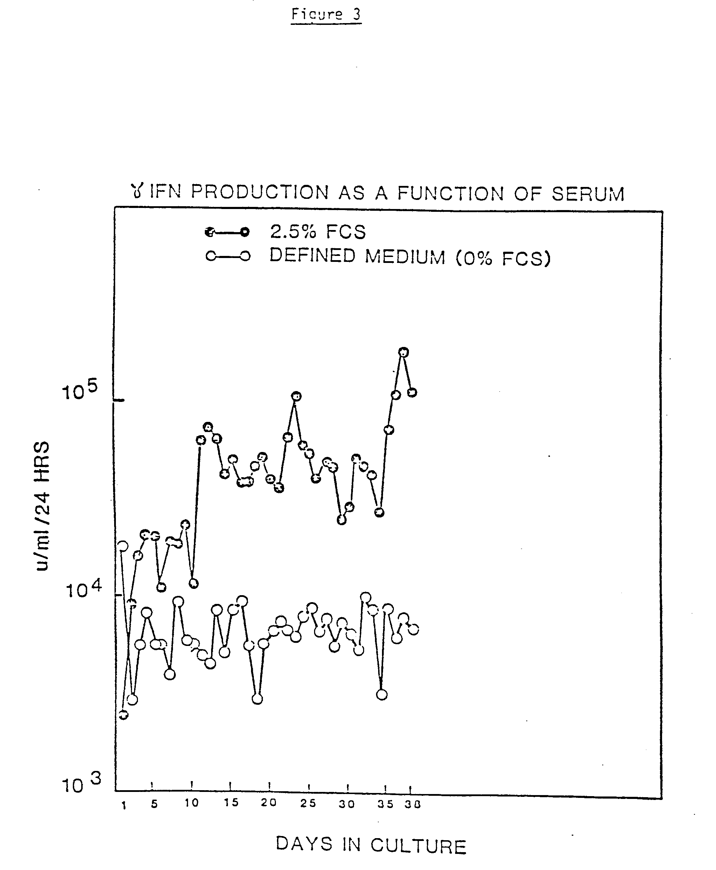

Figure 3 illustrates the requirement for fetal calf serum in long-term expression of recombinant γ-IFN. The production of γ-IFN was examined as a function of the serum concentration in the medium. Duplicate cultures of transformed cells in 60 mm dishes were grown in the presence or absence of FCS, and the culture media was harvested daily and assayed for the presence of biologically active γ-IFN.

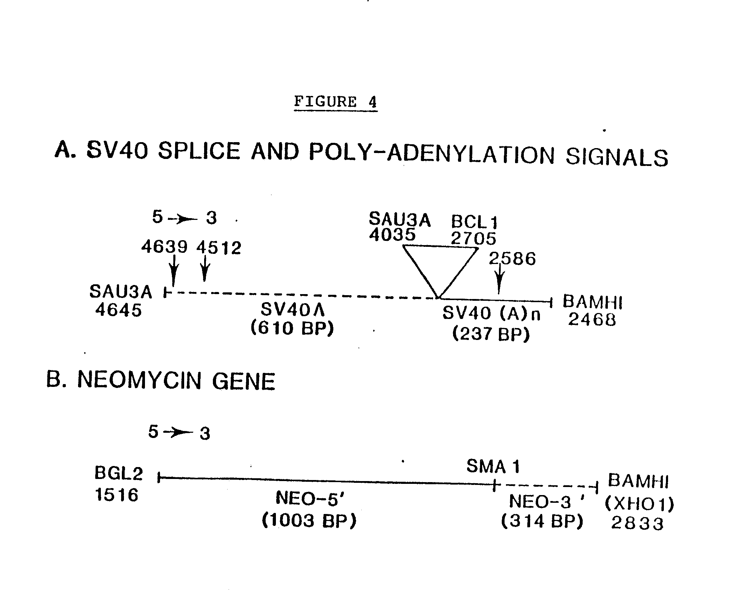

Figure 4 illustrates the physical structure of the SV40 and neomycin genetic elements.

A: SV40 processing elements consist of a fused fragment comprised of two noncontiguous regions of the SV40 genome:
1) A 610 bp region (4645-4034 on the SV40 Map) contains the early intron (66 BP) of the viral genome.
2) The 237 bp fragment (2705-2468 on the SV40 map) contains the polyadenylation signal at position 2586.

B: The neoymcin gene is divided into two functional parts:
1) A 1,003 bp fragment derived from the 5' end of the gene (Bg1II to SmaI) is sufficient to confer resistance to G418.
2) The 3' end region (SmaI to XhoI) is dispensible for the biological activity of the gene. Alone, this 314 bp fragment does not impart resistance to G418.

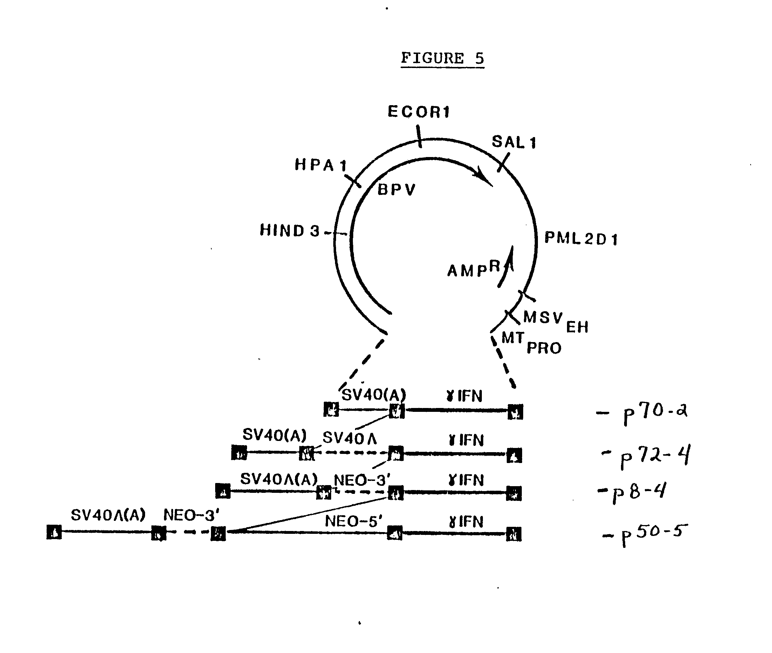

Figure 5 illustrates the physical structure of BPV/gamma-interferon vectors with modified 3' ends. Four vectors were derived from the same parental plasmid containing the following elements (described in a clockwise direction): the complete BPV genome; pML2d1; Maloney murine sarcoma virus enhancer (MSV$_{EH}$); metallothionein promoter - (MT$_{PRO}$ ); and human gamma-interferon cDNA. The constructs differ in the sequences 3' to the gamma-interferon cDNA;

p50-5: Contains the entire neomycin gene (5' and 3' sequences) followed by the SV40 early intron and polyadenylation site.

p8-4: Contains the 3' end of the neoymcin gene followed by SV40 early intron and polyadenylation site.

p72-4: Contains the SV40 early intron and polyadenylation site.

p70-2: Contains the SV40 polyadenylation site.

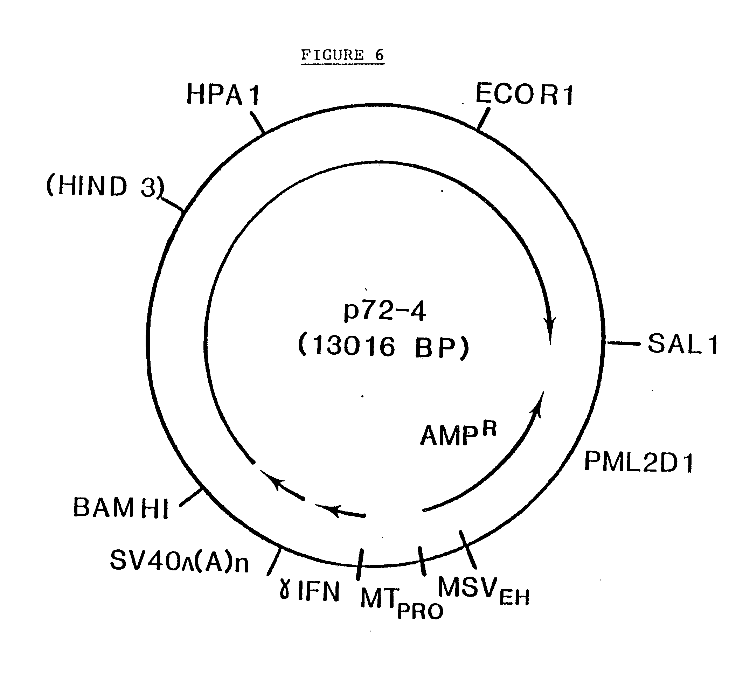

Figure 6 illustrates the physical map of pdBPV-human gamma-interferon (p72-4) DNA. p72-4 is a modified BPV/gamma-interferon plasmid derived from p8-4 after deleting the Neo-3' fragment (314 bp). The plasmid consists of the following structural elements described in a clockwise direction: the complete BPV genome is joined to the pML2d1 sequence containing the pBR322 origin of replication and a functional beta-lactamase gene. The Maloney murine sarcoma virus enhancer (MSV) was originally derived from the proviral LTR as a Hinfl-Xbal fragment (141-525, respectively). Both termini had previously been converted to BamH1 sites with synthetic linkers. The mouse metallothionein I promoter is a Kpnl to Bg1 II fragment of approximately 600 bp. The Kpnl site was previously modified to a Bg1II site to facilitate cloning. Downstream from the promoter is the human gamma-interferon cDNA obtained as a Sau3A fragment which is followed by RNA processing signals derived from SV40 DNA.

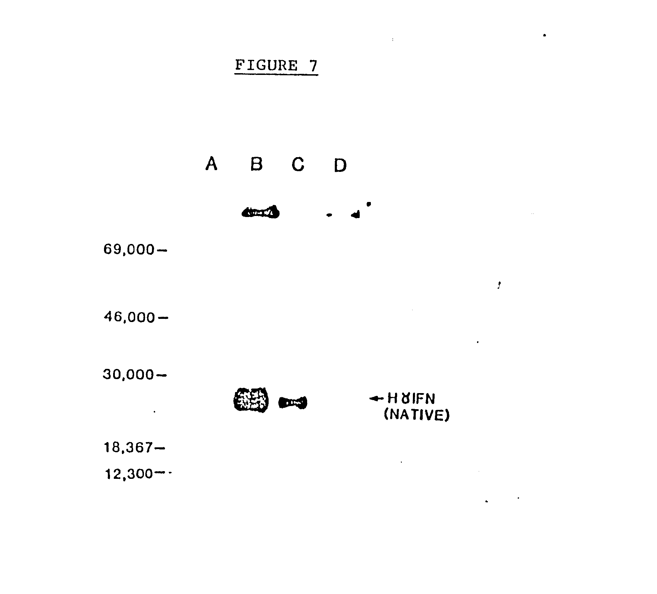

Figure 7 illustrates the immunoprecipitation of extracullular γ-IFN secreted from NS8-4-G-4-transformants. Cells in 100 mm plates were washed with methionine-free medium (GIBCO) containing 2% dialyzed fetal calf serum (FCS) and labeled in 3 ml of the same medium with 400 uCi/ml of 1-$^{35}$-S-methionine (1063.6 Ci/mmol; New England Nuclear) for 4 hours at 37°C. with anti-human gamma interferon serum as described previously. For competition studies, the antiserum was first preincubated with 2 ug of human γ-IFN after which the complex was added was performed on a 10% SDS-polyacrylamide gel after which the gels were treated with En³Hance (New England Nuclear) and fluorographed.

Lane A: Normal goat serum

Lane B: Goat anti-γ-IFN serum

Lane C: Goat anti-γ-IFN serum neutralized with 2 uf of γ-IFN prior to immunoprecipitation.

Lane D: Extracellular medium is from transformants harboring a recombinant DNA in which the γ-IFN cDNA is in a transcriptional direction opposite that of the metallathione in promoter. The sample was treated with goat anti-γ-IFN serum.

DETAILED DESCRIPTION OF THE INVENTION

As used herein, "interferon" denotes human immune interferon or its fragments produced by cell or cell-free culture systems, in bioactive forms having the capacity to influence cellular growth and viral replication as does immune interferon native to the human plasma.

Different alleles of interferon may exist in nature. These variations may be characterized by difference(s) in the nucleotide sequence of the structural gene coding for proteins of identical biological function. In addition, the location and degree of glycosylation as well as other post-translation modifications may vary and will depend to a degree upon the nature of the host and environment in which the protein is produced. It is possible to produce analogs having single or multiple amino acid substitutions, deletions, additions, or replacements. All such allelic variations, modifications, and analogs resulting in derivatives of interferon which retain the biologically active properties of native interferon are included within the scope of this invention.

Expression vectors refer to vectors which are capable of transcribing and translating DNA sequences contained therein, where such sequences are linked to other regulatory sequences capable of effecting their expression. These expression vectors must be replicable in the host organisms or systems either as episomes, bacteriophage, or as an integral part of the chromosomal DNA. One form of expression vector which is particularly suitable for use in the invention is bovine papillomavirus (PBV) DNA.

The phrase shuttle vector means the vector contains the necessary replication regions (replicons) to allow replication in two distinct hosts. The shuttle vectors of this invention are capable of replication in E. coli, a prokaryotic organism due to the presence of a bacterial replicon contained in the plasmid PML2D1, itself a derivative of plasmid pBR322 as well as capable of replication in an PBV susceptible eukaryotic cell line due to the BPV replicon.

The molecularly cloned bovine papillomavirus 1 DNA as well as a cloned 69% subgenomic fragment of the bovine papillomavirus 1 genome are very efficient in inducing transformed foci in susceptible mouse cell [Howley, P.M., et al. 1980, in M. Essex, et al., (ed.), Viruses in Naturally Occurring Cancers, (Cold Spring Harbor Laboratory; Cold Spring Harbor, New York) p. 233-247 and Lowry, D.R., et al., Nature (London) (1980) 287:72]. Bovine papillomavirus-transformed cells contain multiple copies (10 to 120 per cell) of the viral DNA. These copies exist exclusively as unintegrated extrachromosomal molecules (Law, et al., supra, thus, offering a natural means of amplifying foreign DNA sequences which are covalently linked to the bovine papillomavirus-transforming segment. Since integration of the viral genome does not occur, - (Law et al., supra), the physical contiguity of the "passenger" DNA segment should be preserved. The transformed phenotype provides a marker for selecting those cells that have incorporated the foreign DNA segment; thus, any cell line susceptible to bovine papillomavirus transformation is a potential recipient. Bovine papillomavirus-transformed cells grow faster than their nontransformed counterparts. This should facilitate the large-scale production of cells containing the exogenous gene and possibly, therefore, the gene product.

Recombinant vectors and methodology disclosed herein are suitable for use in host cells covering a wide range of prokaryotic and eukaryotic organisms. In general, of course, prokayotes are preferred for the cloning and amplification of DNA sequences and in the construction of vectors. For example, E. coli K12 strain HB101 - (ATCC No. 33694), is particularly useful. Of course, other microbial strains may be used. Vectors containing replication and control sequences which are derived from species compatible with the host cell or system are used in connection with these hosts. The vector ordinarily carries an origin of replication, as well as genetic characteristics capable of providing phenotypic selection in transformed cells. For example, E. coli can be transformed using the vector pBR322, which contains the promoters and genes for ampicillin and tetracycline resistance - [Bolivar, et al., Gene, 2:94 (1977)].

These antibiotic resistance genes provide a means of identifying transformed cells. The expression vector may also contain control elements which can be used by the vector for expression of its own proteins. Common prokaryotic control elements used for expression of foreign DNA sequences in E. coli include the promoters and regulatory sequences derived from the β-galactosidase and tryptophan (trp) operons of E. coli, as well as the pR and pL promoters of bacteriophage lambda. Combinations of these elements have also been used (e.g., TAC, which is a fusion of the trp promoter with the lactose operator). Other promoters have also been discovered and utilized, and details concerning their nucleotide sequences have been published enabling a skilled worker to combine and exploit them functionally.

In addition to microorganisms, cultures of cells derived from multicellular organisms may also be used as hosts. In principle, any such cell culture is workable, whether from a vertebrate or invertebrate source. However, interest has been greatest in vertebrate cells, and propagation of vertebrate cells in culture (tissue culture) has become a routine procedure in recent years. Examples of such useful hosts are the mouse L cells, VERO, HeLa, mouse C127, Chinese hamster ovary (CHO), W138, BHK, COS-7, and MDBK cell lines. Expression vectors for such cells ordinarily include an origin of replication, a promoter located upstream of the gene to be expressed, along with any RNA splice sites, polyadenylation site, and transcription termination sequence.

For use in mammalian cells, the control functions on the expression vectors are often provided by viral derived sequences. For example, commonly used promoters are derived from polyoma, Adenovirus 2, and most frequently, Simian Virus 40 (SV40). Further, it is also possible and often desirable, to utilize promoter or control sequences naturally associated with the desired gene sequence, provided such control sequences are compatible with the host system. To increase the rate to transcription, eukaryotic enhancer sequences can also be added to the construction. These sequences can be obtained from a variety of DNA viruses such as SV40, polyoma or oncogenic retroviruses such as the mouse sarcoma virus and mammary tumor virus.

A mammalian origin of replication is usually part of the viral vector used as a vehicle, such as that provided by SV40 or other viral sources, or may be provided by the host cell chromosomal replication mechanism. If the vector is integrated into the host cell chromosome, the latter is often sufficient.

Host cells can prepare interferon proteins which can be a variety of chemical compositions. The protein is produced having methionine as its first amino acid (present by virtue of the ATG start signal codon naturally existing at the beginning of the structural gene or inserted before a segment of the structural gene). The protein may also be intra- or extracellularly cleaved, giving rise to the amino acid which is found naturally at the amino terminus of the protein. The protein may be produced together with either its signal polypeptide or a conjugated protein other than the convention signal polypeptide, the signal polypeptide of the conjugate being specifically cleavable in an intra or extracellular environment. Finally, interferon may be produced by direct expression in mature form without the necessity of cleaving away any extraneous polypeptide.

The signal sequence used herein is the γ-IFN signal sequence described by Devos, et al. - (Nucleic Acid Res. 10:2487-1501 (1982) and Gray, et al. (Nature 295:503-508 (1982)) which includes the coding region for an additional three amino acids (lys, tyr, cys) 3' to the region reported. This modified sequence is a unique signal sequence construction and is useful for the expression and export of a variety of other protein products in addition to IFN-γ for which open reading found sequences are available.

Recombinant host cells refer to cells which have been transformed with vectors constructed using recombinant DNA techniques. As defined herein, immune interferon is produced as a consequence of this transformation. Immune interferon or its fragments produced by such cells are referred to as "recombinant immune interferon".

Recombinant and Screening Methodology

The procedures below are but some of a wide variety of well established procedures to produce specific reagents useful in the process of this invention. Double-stranded cDNA (ds-cDNA) is inserted into the expression vector by any one of many known techniques. In general, methods, etc., can be found in Maniatis, supra, and Methods In Enzymology, Volumes 65 and 68 (1980); and Volume 100 and 101 (1983). In general, the vector is linearized by at least one restriction endonuclease, which will produce at least two blunt or cohesive ends. The ds-cDNA is ligated with or joined to the vector insertion site.

If prokayotic cells or other cells which contain substantial cell wall material are employed, the most common method of transformation with the expression vector is calcium chloride pretreatment as described by Cohen, R.N., et al., Proc. Natl. Acad. Sci. USA, 69:2110 (1972). If cells without cell wall barriers are used as host cells, transfection is carried out by the calcium phosphate coprecipitation method described by Graham and Van der Eb, Virology, 52:456 (1973). Other methods for introducing DNA into cells such as nuclear injection or protoplast fusion, have also been successfully used. The organisms are then cultured on selective media and proteins for which the expression vector encodes are produced.

Clones containing the entire gene for immune interferon are identified in a semi-micro C.P.E. inhibition assay using WISH cells as the indicator cells and EMC as the challenge virus. This method of identification requires that the ds-cDNA be inserted into a vector containing appropriate regulatory nucleic acid sequences adjacent to the insertion site. These regulatory sequences initiate transcription and translation of those ds-cDNA molecules inserted in the vector. Those clones containing interferon cDNA sequences correctly positioned relative to the regulatory sequences synthesize part or all of the interferon protein. Restriction endonuclease digestion and nucleotide sequencing techniques are used to determine the sequence of amino acids encoded by the cDNA fragments.

The invention may be more fully appreciated by reference to the following Examples which in no event should be construed to limit the invention.

EXAMPLE 1

Isolation of the Recombinant Clone

The gamma interferon cDNA clone employed herein was obtained as follows. Briefly, size fractionated mRNA was isolated from human peripheral blood lymphocytes that had been induced with A23187 and mezzarine (I.A. Braude, Biochemistry, 23:5603, 1984). The mRNA was converted into double-stranded cDNA and cloned into the PstI site of pBR322 by G-C tailing using standard procedures. An 843 base pair Sau3A fragment containing the gamma interferon signal peptide, coding region, and translation termination codon, was cloned into a BPV derivative vector as shown in Figure 1. The resulting construct contained gamma interferon sequences placed downstream from a mouse metallothienein 1 promoter, MSV enhancer sequence, and also contained the SV40 small t splice sites and polyadenylation site. The vector also contains 314 b.p. between the 3' end of the γ-IFN cDNA and the SV40 elements. The 314 b.p. are derived from the 3' ends of the neomycin gene (See Figure 4B).

C127 mouse cells were transformed using the calcium phosphate coprecipitation method (Graham and van der Eb, supra) followed by enhancement with 25% dimethylsulfoxide (Stowe and Wilkie, J. Gen. Virol., 33:447, 1976) as described previously - (Sarver, et al., Mol. Cell Biol., 1:486, 1981). Phenotypically transformed foci were isolated and established as cell lines. Single cell cloning was achieved by plating at limited dilution into 96 well plates (Co-star), incubating at 37°C., identifying wells containing a single cell, and propagating these cells into established lines.

As an initial characterization of the foci, tissue culture media from transformed cells derived from 16 individual foci was collected and assayed for the presence of biologically active gamma interferon. The biological assay specifically measured a reduction in the cytopathic effect of EMC on human WISH cells. The results in Figure 2A demonstrate a large variation in the amount of biologically active gamma interferon produced by different foci. However, when the analysis was performed on 24 independent single-cell clones obtained from a single transformed focus (number 8-4-G), the amount of biologically active gamma interferon produced differed over a much smaller range (Figure 2B).

Having identified gamma interferon biological activity BPV transformed cells, the nature of the polypeptide product was investigated. Cells were labelled with $^{35}$S-methionine, extracellular media was harvested and immunoprecipitated with a polyclonal goat anti-gamma interferon serum. The precipitated proteins were then analyzed on SDS polyacrylamide gels as described elsewhere - (Sarver, et al., Mol. Cell. Biol. 1:486-496 (1981)). Analysis of BPV transformed cells demonstrated the presence of a prominent band identical in its electrophoretic mobility to native gamma interferon (Figure 7). This band was absent from the media obtained from cells transformed by BPV constructs containing gamma interferon cDNA inserted in the opposite orientation. In a competitive binding study, samples were incubated with antiserum previously neutralized with 2 μg of gamma interferon. Under these conditions, a significant reduction of the amount of gamma interferon-like protein that was immunoprecipitated.

The production of crude gamma interferon was subsequently examined as a function of the serum concentration in the medium. Duplicate cultures of transformed cells were grown in the presence or absence of fetal calf serum. The culture media was harvested daily and assayed for the presence of biologically active gamma interferon. The data in Figure 3 demonstrated that approximately 10-fold more gamma interferon is produced in cells maintained in the presence of 2.5% fetal calf serum when compared with cells maintained in the absence of serum.

Purification of Recombinant Human Gamma Interferon

In order to obtain sufficient material for biochemical characterization, approximately 50 liters of crude tissue culture medium was collected and used for large-scale protein purification. The purification procedure used was essentially that described by Braude (supra) for the purification of natural gamma interferon. The protocol consists of essentially 5 steps that are summarized in Table 1.

## TABLE 1

### PURIFICATION OF RECOMBINANT HUMAN GAMMA INTERFERON BY SEQUENTIAL CHROMATOGRAPHY

| Step[†] | Volume (ml) | IFN (IU) X $10^{-8}$ | Protein (mg) | Specific Activity (IU/mg) | Total Fold Purification | Total % Recovery |
|---|---|---|---|---|---|---|
| CPG | | | | | | |
| Input | 50,000 | 7.85 | 81,500 | $9.63 \times 10^3$ | -- | -- |
| Output | 2,300 | 7.85 | 3,772 | $2.08 \times 10^5$ | 22 | 100 |
| ConA/S | | | | | | |
| Output | 1,370 | 7.85 | 356 | $2.20 \times 10^6$ | 228 | 100 |
| H/S | | | | | | |
| Output | 65 | 6.64 | 115 | $5.77 \times 10^6$ | 599 | 85 |
| Sephadex G-100 | | | | | | |
| Output | 92 | 6.64 | 48 | $1.38 \times 10^7$ | 1,433 | 85 |
| CM-BGA | | | | | | |
| Input[*] | 36[*] | 3.22[*] | 11[*] | $2.93 \times 10^{7}$[*] | -- | -- |
| Output | 60 | 2.43 | 4 | $6.08 \times 10^7$ | 6,314 | 77[+] |

[†] The abbreviations used are CPG, Controlled Pore Glass; ConA/S, concanavalin A-Sepharose; H/S, heparin Sepharose; CM-BGA, carboxymethyl-Bio-Gel-agarose.
[*] Selected fractions from the Sephadex G-100 output were processed on CM-BGA.
[+] Recovery assuming all the Sephadex G-100 was processed.

Material obtained from the entire 50 liters of media was processed through the Sephadex G-100 step. This resulted in an 85% recovery of the gamma interferon activity present in the starting material. Approximately one-third of the G-100 material was then further processed on CM-Biogel. Peak points of the Biol-Gel column had a specific activity of $1.2 \times 10^8$ IU per mg. The specific activity of gamma interferon has been reported to be between 1.0 and $2 \times 10^8$ IU/mg, consequently, the recombinant material purified by the protocol approaches that of the pure human gamma interferon.

The recombinant protein is biologically indistinguishable from nature $\gamma$-IFN in that it confers antiviral protection on appropriate host cells, induces the expression of class II major histocompatibility - (MHC) antigens in HeLa and endothelial cells and is neutralized by anti-human-γ-IFN serum. Its identity with authentic human γ-IFN is further illustrated by the fact that a monoclonal antibody (provided by Rebesh Rubin, New York Blood Center) which recognized nature γ-IFN isolated from included peripheral blood leukocytes, but not bacterially produced recombinant human γ-IFN, completely neutralizes the γ-IFN obtained from the engineered C127 cells.

Greater than 80% of the recombinant protein is found extracellularly, indicating efficient transport across the cell membrane. The reported amino acid sequence of γ-IFN accounts for a peptide of 17,000 daltons. The apparent size of 22,000-25,000 daltons of crude (Figure 4) purified γ-IFN, coupled with the observation that it binds to the ConA sepharose column, strongly suggests that it contains carbohydrate moieties. Thus, post-translational modifications of the γ-IFN protein, including removal of the signal peptide and glycosylation of the protein appear to be faithfully carried out in transformed C127 cells.

EXAMPLE II

In an attempt to increase the level of expression the DNA region adjacent to the 3' encoding end of the γ-IFN genetic region was subjected to various modifications. With refernce to Figures 1 and 4, the detail of the region is indicated by the enlarged insert. The region of modification comprises an inactive fragment of the neomycin resistance gene, a region of SV40 DNA containing 1) small t-splice sites which have been implicated in the post-transcriptional modification of mRNAs, as well as 2) a polyadenylation site.

As used herein the phrase posttranscriptional regulatory includes sequences involved with mRNA processing events such as the small t-splice regions and poly A addition sequences described above.

Two of these elements 3' to the gamma interferon gene theoretically are dispensible for gene expression: A 314 bp fragment representing only the 3' portion of the neomycin gene which does not impart resistance to neomycin on the host cell; and a donor acceptor site derived from the SV40 small "t" intron. Since the gamma interferon insert is already a processed cDNA, splicing should not be required for gene expression. Figure 4 depicts the structural features of these elements.

In order to minimize the overall size of the DNA vector, additional vectors were constructed which differed only in the region 3' to gamma interferon cDNA (Figure 5), and assessed their potential for directing high-level gene expression. These results, which are summarized in Table 2, demonstrate the following.

Sequences derived from the neomycin gene interfere with gamma interferon expression when placed between the 3' terminus of the insert and the RNA processing signals. Thus, p8-4, containing the 3' portion of the neomycin gene at this position, induces only 10-20% the level of gamma interferon as does p72-4 from which the neomycin sequences had been removed. This interference with gene expression is even more dramatic when the entire neoymcin gene (5' and 3' portions) is placed downstream of the gamma interferon cDNA. In this case, no gamma interferon biological activity was detected in most of the lines tested (p50-2, Table 2). Surprisingly, cells containing p50-2 DNA are resistant to G418, indicating that the neomycin gene is fully functional.

The presence of a SV40 small "t" intron has an augmenting effect on gamma interferon cDNA expression. Thus, p70-21, deleted of SV40 splicing signals is only 50% as efficient is splicing gamma interferon as p8-4, the original vector.

This result was completely unexpected since the γ-IFN is a cDNA insert, the necessity of region encoding splicing elements should not be required. The reason for the enhancing ability of this region is unknown.

Several cell lines harboring the modified p72-4 (depicted in Figure 6) were established, some capable of secreting as much as ten-fold high levels of gamma interferon that previous lines. Extensive biochemical characterization of one such line - (NS72-4-9-6) has demonstrated that the plasmid DNA is maintained as an episome at high-copy number, the gamma interferon specific RNA is of the correct size and that the secreted protein is a glycosylated monomer 22,000-26,000 d in size. Greater than 95% of the foreign protein was secreted outside the cell proper.

The cell lines have been in culture for at least four months and are stable with regard to the level of human gamma interferon expression.

## TABLE 2

### EFFECT OF 3' SEQUENCES ON GAMMA-INTERFERON EXPRESSION

| DNA | 3' Description | U/ml/$10^6$ cells | No. Analyzed | U Range |
|-----|----------------|-------------------|--------------|---------|
| p70-21 | SV40 (A)n | 8,370 | 20 | 100-30,500 |
| p72-4 | SV40 ∧ (A)n | 119,117 | 23 | 39,800-450,30 |
| p8-4 | NEO-3' SV40 ∧ (A)n | 15,542 | 13 | 2,820-64,890 |
| p50-2 | NEO 5' NEO 3' SV40∧(A)n | 2,210 | 20 | <30-5,800 |

Deposit of Strains Useful in Practicing the Invention

A deposit of biologically pure cultures of the following strains was made with the American Type Culture Collection, 12301 Parklawn Drive, Rockville, Maryland, the accession number indicated was assigned after successful viability testing, and the requisite fees were paid. Access to said culture will be available during pendency of the patent application to one determined by the Commissioner to be entitled thereto under 37 C.F.R. §1.14 and 35 U.S.C. §122. All restriction on availability of said culture to the public will be irrevocably removed upon the granting of a patent based upon the application and said culture will remain permanently available for a term of at least five years after the most recent request for the furnishing of a sample and in any case for a period of at least 30 years after the date of the deposit. Should the culture become nonviable or be inadvertently destroyed, it will be replaced with a viable culture(s) of the same taxonomic description.

| Strain/Plasmid | ATCC No. | Deposit Date |
|----------------|----------|--------------|
| pdBPV-H- Υ -IFN (8-4) | 53087 | April 9, 1985 |
| pdBPV-H- Υ -IFN (p72-4) | 53277 | Sept. 30, 1985 |

## Claims

1. A bovine papillomavirus expression vector capable of replication in a prokaryotic host and replication and expression of non-native genetic information in an eukaryotic host comprising a prokaryotic replicon, a eukaryotic replicon, a prokaryotic promoter, a eukaryotic promoter, at least two dominant selectable markers, a signal sequence, and an enhancer element.

2. The vector according to Claim 1 wherein said prokaryotic replicon is derived from pBR 322; wherein said eukaryotic replicon is derived from bovine papillomavirus BPV-1; wherein said prokaryotic promoter is the β-lactamase promoter; wherein said eukaryotic promoter is the mouse metallothienein I gene promoter; wherein said selectable markers confer ampicillin resistance and phenotype transforming ability; wherein said signal sequence is the IFN-γ signal sequence; wherein said enhancer element is the enhancer element from the Maloney murine sarcoma virus LTR.

3. The vector according to Claim 1 or 2 which includes post-transcriptional regulatory sequences and a neomycin resistance gene fragment.

4. The vector according to Claim 3 wherein said regulatory sequences are the SV40 splicing sequence and the poly A addition sequence.

5. The vector according to any of the Claims 1-4 having the identifying characteristics of p8-4 and derivatives thereof.

6. The vector according to any of the Claims 1-5 including a non-native DNA segment being in an orientation with said promoters, signal sequence, and post transcriptional regulatory sequences such that in the host it expresses a non-native product encoded by said non-native DNA segment.

7. The vector according to Claim 6 wherein said non-native segment is human IFN-γ.

8. The vector according to Claims 6 or 7 wherein said vector has the identifying characteristics of pdBPV-γ INF. (ATCC 53087)

9. A host transformed by the vector of any of the Claims 1-8.

10. The vector according to Claim 3 wherein the neomycin resistance gene fragment has been deleted.

11. The vector according to Claim 10 wherein the vector has the characteristics of p72-4.

12. The vector according to Claims 10 or 11 including a non-native DNA segment being in an orientation with said promoters signal sequence, and posttranscriptional regulatory sequences such that in the host it expresses a non-native product encoded by said non-native DNA segment.

13. The vector according to Claim 12 wherein said non-native segment is human IFN-γ.

14. The vector according to Claims 12 or 13 wherein the vector has the identifying characteristic of ATCC 53277.

15. The host transformed by the vector of any of the Claims 10-14.

16. The host according to Claims 9 or 15 wherein the host is C127 mouse cells.

17. In a process for producing human interferon comprising providing a replicable expression vector capable of expressing the DNA sequence encoding immune interferon in a suitable host, transforming said host, and maintaining said transformed host under conditions permitting the expression of said interferon the improvement which comprises the employing of vector pdBV γ-IFN as the replicable expression vector.

0 198 386

FIGURE 1

PHYSICAL STRUCTURE OF γ-IFN cDNA CLONE 118 E9

Figure 2a

γ IFN PRODUCTION IN ISOLATED FOCI
u/ml /$10^5$CELLS/24 HOURS

FOCI NUMBER

Figure 2b

CLONE NUMBER

Figure 3

ɤ IFN PRODUCTION AS A FUNCTION OF SERUM

FIGURE 4

## A. SV40 SPLICE AND POLY-ADENYLATION SIGNALS

## B. NEOMYCIN GENE

0 198 386

## FIGURE 5

FIGURE 6

FIGURE 7

A    B    C    D

69,000—

46,000—

30,000—

←H ඪIFN
(NATIVE)

18,367—

12,300—·